# EUROPÄISCHE PATENTANMELDUNG

(11) **EP 2 587 261 A1**
(43) Veröffentlichungstag der Anmeldung: **01.05.2013**
(21) Anmeldenummer: 11186472.4
(22) Anmeldetag: 25.10.2011
(51) Int. Cl.: G01N 33/15, G01B 11/02, G01N 3/40

(54) **Tablettenteststation**

(71) Anmelder: Pharmatron AG, 3600 Thun (CH)
(72) Erfinder: Boss, Thomas, 3661 Uetendorf (CH); Herrmann, Holger, 3600 Thun (CH)
(74) Vertreter: Patentbüro Paul Rosenich AG

(57) **Zusammenfassung**

Tablettenteststation, umfassend mindestens eine Aufnahme zur Übernahme von Tabletten von einem Auslass einer Beschickungsvorrichtung und mindestens ein Prüfmittel zum Prüfen, Testen und/oder Messen der Tabletten. Es ist zudem eine Hebevorrichtung (20) zum Verfahren der Aufnahme (15) relativ zur Beschickungsvorrichtung (13) vorgesehen.

## Beschreibung

Die Erfindung bezieht sich auf eine Tablettenteststation der im Oberbegriff des Anspruchs 1 genannten Art, auf eine Maschine zum Testen von Tabletten mit der Tablettenteststation gemäss Anspruch 12 und auf ein Verfahren zum Testen von Tabletten in einer besagten Tablettenteststation gemäss Anspruch 13.

In Forschung und Industrie sind solche Tablettentestgeräte im Einsatz, um den sehr hohen Qualitätsanforderungen vor allem im pharmazeutischen Bereich Genüge zu tun. Diese Geräte sind manuell bedienbar, halb- oder vollautomatisch. Sie messen z.B. Gewicht, Dicke, Durchmesser, beziehungsweise Länge und Breite sowie Härte, also Bruchfestigkeit und Bruchverhalten von Tabletten mit mechanischen, elektronischen, optischen, chemischen oder akustischen Verfahren. Da Tabletten in unterschiedlichster Gestalt vorliegen, ist die relativ zum Messaggregat optimale und sichere Positionierung der Tablette im jeweiligen Verfahren eine grosse Herausforderung für den Entwickler und Anwender solcher Tablettentestgeräte. Dies gilt insbesondere, wenn an einer Tablettenteststation verschiedene Prüfverfahren durchgeführt werden, die unterschiedliche Positionierungen der Tablette verlangen.

Im Stand der Technik sind Teststationen zum Testen von Tabletten, umfassend eine Aufnahme zur Übernahme von Tabletten von einem Auslass einer Beschickungsvorrichtung und mindestens ein Prüfmittel zum Prüfen, Testen und/oder Messen der Tabletten beschrieben.

In der EP 1 531 317 A1 ist ein Verfahren zur Durchführung von Dicke- und Härtemessungen an Prüfkörpern, insbesondere Tabletten, und ein Drehteller hierfür beschrieben. Hierin wird eine Teststation mit einem Drehteller als Aufnahme von Tabletten beschrieben, die auf demselben einem Messverfahren zur Ermittlung von Länge und Durchmesser unterzogen werden. Mit einem Aufgreifmittel werden die Tabletten anschliessend einzeln vom Drehteller abgehoben und in einer anderen Teststation abgelegt.

Dieses Verfahren hat mehrere Nachteile: Es gibt unterschiedlich ausgestaltete Teststationen für verschiedene Testverfahren. Dementsprechend gibt es auch verschiedene Zuführungen, beziehungsweise Übergaben. Jede dieser Teststationen braucht Platz und verursacht zusätzliche Kosten. Jede Übergabe kostet Zeit.

Aus der DE 102008035830 A1 ist eine Vorrichtung zur Durchführung eines Härtetests und einer Längen-, Breiten- und Höhenmessung eines Prüfkörpers, insbesondere einer Tablette, in einer einzigen Teststation beschrieben. Die Vorrichtung weist eine Grundplatte als Aufnahme zur Übernahme für die Tablette aus einer Beschickungsvorrichtung und Mittel zum Prüfen, Testen und/oder Messen der Tablette auf. Die Teststation wird gezeigt mit einer Druckmessdose, einer Druckplatte, einem Anschlagstück und einer Lasermesseinrichtung, bei der auf der Grundplatte das Anschlagstück, mit einem Schieber verbundene Schiebeleisten und eine Spindel zum Verschieben von Ausrichtzangen angeordnet sind. Es sind dazu Ausrichtzangen beschrieben, die muldenartige Öffnungen mit einer Aussparung für das Positionieren der Tablette sowie einen Messspalt aufweisen. Auf der Grundplatte ist eine in Richtung des Anschlagstückes verschiebbare Tischplatte für die Tablette vorgesehen. Die Druckmesssonde ist mit dem Schieber und der Druckplatte verbunden.

Diese Lösung ist aufwändig und weder für alle Ausgestaltungen von Tablettenteststationen noch für alle Arten der Zuführung von Tabletten geeignet.

Der Stand der Technik lässt sich dahingehend zusammenfassen, dass eine unterschiedliche Positionierung der Tabletten in Tablettenteststationen vor und/oder während der verschiedenen Arbeitsgänge unzureichend oder nur mit erheblichem Aufwand erreicht wird. Wird die Tablette - oft durch ihre spezielle Form bedingtnicht für jedes Mess-, beziehungsweise Testverfahren in die jeweils richtige Position gebracht, ist das Mess-, beziehungsweise Testergebnis mangelhaft. Jedes Nachpositionieren der Tablette, um ein optimales Testergebnis zu erhalten, führt zu Verzögerungen im Verfahrensablauf und damit zu geringerer Wirtschaftlichkeit der Tablettenteststation. Speziell problematisch ist das Testen aussergewöhnlicher Tablettenformen, weil sich diese nicht durch ein universelles Verfahren in die erforderliche Position bringen lassen.

Aufgabe der Erfindung ist es daher, die vorgenannten Nachteile zu überwinden und eine konstruktiv einfachere und günstigere Tablettenteststation zur Verfügung zu stellen, in der Tabletten jeder Form mit hoher Genauigkeit positioniert und verschiedene Testverfahren mit höchstmöglicher Geschwindigkeit durchgeführt werden können.

Die Aufgabe ist durch das Merkmal des unabhängigen Anspruchs gelöst. Vorteilhafte Weiterbildungen sind in den abhängigen Patentansprüchen und in den Figuren dargelegt.

Gemäss der Erfindung ist eine Hebevorrichtung zum Verfahren der Aufnahme zur Übernahme der Tabletten aus der Beschickungsvorrichtung relativ zur derselben vorgesehen, ausgebildet nach Art eines Liftes.

Besonders vorteilhaft ist diese Hebevorrichtung in Kombination mit besonders ausgebildeten Beschickungsvorrichtungen und/oder mit einer verschwenkbaren Aufnahme für Tabletten, wie sie in einer Anmeldung mit dem internen Aktenzeichen D8PEP derselben Anmelderin mit demselben Anmeldedatum wie dem der hier vorliegenden Anmeldung gezeigt wird. Diese Anmeldung gilt als integrierender Bestandteil der hier vorliegenden Anmeldung.

Die Hebevorrichtung ist mit wirtschaftlichen wie technischen Verbesserungen verbunden: Sie erlaubt eine dem Prozess angepasste exakte Positionierung jeder vorstellbaren Tablette innerhalb kürzester Zeit und ist im Prinzip bei jedem Testverfahren und bei jeder Art der Beschickung automatischer Art vorstellbar. Als automatische Beschickung wird hierbei jegliche Art der Beschickung verstanden, die nicht rein manuell erfolgt. Ein besonderer Vorteil der Erfindung liegt darin, die Aufnahme für die Tablette sehr nahe an den Auslass der Beschickungsvorrichtung verfahren zu können, so dass die zu übergebende Tablette nicht, wie im Stand der Technik, unkontrolliert auf die Aufnahme fällt und an irgendeiner Stelle liegenbleibt, sondern bereits für das, vorzugsweise erste, Testverfahren positioniert ist. Ein weiterer Vorteil der Erfindung liegt darin, dass die Tablette möglichst nahe an der festen Backe positioniert werden kann. Somit ist beim anschliessenden Messen kein zeitaufwändiges Verschieben der Tablette erforderlich und das Risiko eines Verdrehens der Tablette kann ausgeschlossen werden.

Die Hebevorrichtung hat vorteilhaft einen Motor, der besonders vorteilhaft von einer Steuerung gesteuert wird, um die schnelle und exakte Positionierung zu gewährleisten.

In einer bevorzugten Ausführung ist das Mittel zum Prüfen, Testen und/oder Messen der Tabletten als Längen- und/oder Breiten- und/oder Dickenmessvorrichtung ausgebildet, das vorzugsweise mindestens eine Positionierfläche zum Positionieren der Tablette auf der Aufnahme aufweist.

Besonders bevorzugt ist dieses Mittel zum Prüfen, Testen und/oder Messen ein optisches, da dieses besonders schnell und exakt arbeitet.

In einer bevorzugten Ausführung ist das Mittel zum Prüfen, Testen und/oder Messen als Bruchfestigkeitsmessvorrichtung und/oder Vorrichtung zum Testen des Bruchverhaltens ausgebildet.

In einer besonders bevorzugten Ausführung umfasst die

Bruchfestigkeitsmessvorrichtung eine Bruchvorrichtung, die mindestens eine Positionierfläche zum Positionieren der Tablette aufweist. Die Nutzung von zumindest Teilen von Mitteln zum Prüfen, Testen und/oder Messen als Positionierflächen reduziert die Anzahl zusätzlich benötigter Positionierflächen beispielsweise an der Aufnahme.

In einer bevorzugten Ausführung weist die Aufnahme mindestens eine Positionierfläche zum Positionieren der Tablette auf, die in einer besonders bevorzugten Ausführung als Umlenkung ausgebildet ist. Diese erlaubt eine einfache und schnelle Positionierung der Tablette.

In einer bevorzugten Ausführung ist die Aufnahme als mindestens eine Klappe ausgebildet. Diese kann ebenso zur Positionierung der Tablette für ein Testverfahren verschwenkt werden wie auch zur Aussscheidung der Tablette aus der Tablettenteststation.

In einer bevorzugten Ausführung sind bei der Anordnung von mehreren Mitteln zum Prüfen, Testen und/oder Messen der Tabletten diese in einer Ebene angeordnet. Bei dieser Anordnung ist die Tablettenteststation in der Lage, die Testverfahren ohne Unterbrechung und damit mit der grösstmöglichen Geschwindigkeit durchzuführen.

Mit Vorteil wird eine erfindungsgemässe Tablettenteststation in einer Maschine zum Testen von Tabletten eingesetzt, die mehrere, bevorzugterweise unterschiedliche Tablettenteststationen und/oder andere Aggregate wie eine Beschickungsvorrichtung umfasst.

Das bevorzugte Verfahren zum Testen von Tabletten mit einer Tablettenteststation oder mit einer Maschine mit einer erfindungsgemässen Tablettenteststation umfasst ein Verfahren der Aufnahme zur Übergabe der Tablette mittels der Hebevorrichtung in eine Position benachbart der Beschickungsvorrichtung, eine Übergabe der Tablette von der Beschickungsvorrichtung an die Aufnahme und anschliessend ein Verfahren der Aufnahme in mindestens eine zweite Position zur Durchführung von Tests. Unter Tests sollen hier auch Prüfungen und Messungen verstanden werden.

Mit grösstem Vorteil verfährt die Hebevorrichtung die Aufnahme zur Übergabe der Tablette so in die erste Position, dass der Abstand der Aufnahme vom Auslass der Beschickungsvorrichtung kleiner als die Dicke der jeweils zu prüfenden Tablette ist, also möglichst nah. So ist es der Beschickungsvorrichtung möglich, je nach Bauart derselben und Gestalt der Tablette, die Tablette in exakter Ausrichtung an einer vordefinierten Stelle auf der Aufnahme zu positionieren. Um das diesbezüglich beste Ergebnis zu erzielen, ist in einer bevorzugten Ausführung der Maschine, umfassend eine Tablettenteststation die Beschickungsvorrichtung mit Auslässen versehen, die eine je definierte Positionierung ermöglichen.

Mit der Hebevorrichtung können Übergabe und das oder die anschliessenden Testverfahren in der Weise und insbesondere auf der Höhe innerhalb der Maschine, umfassend eine Tablettenteststation, beziehungsweise Teststation durchgeführt werden, wie ursprünglich konstruktiv vorgesehen. Die Hebevorrichtung stellt also eine relativ einfache und - im Vergleich zu einer Neukonstruktion einer Tablettenteststation, beziehungsweise einer ganzen Maschine, umfassend eine Tablettenteststation, zu Optimierungszwecken - kostengünstige Verbesserung der konstruktiv bedingten Nachteile von Tablettentestvorrichtungen mit Beschickungsvorrichtungen dar, die insbesondere auch nachträglich in eine Maschine, umfassend eine Tablettenteststation eingebaut werden kann.

In einer vorteilhaften Ausführung wird die Tablette zum Abschluss des Verfahrens aus der Tablettenteststation ausgeschieden, sei es, um einem weiteren Testverfahren unterzogen zu werden, sei es, um entsorgt zu werden.

Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile.

Es zeigen dabei
- Fig. 1 -: ein erstes erfindungsgemässes Ausführungsbeispiel einer Tablettenteststation in schematischer Darstellung,
- Fig. 2 - 5: ein erfindungsgemässes Ausführungsbeispiel einer Tablettenteststation in verschiedenen Phasen des darin durchgeführten Verfahrens.

Zur Vereinfachung, aber keineswegs auf diese spezielle Tablettenteststation eingeschränkt, wird die erfindungsgemässe Anordnung in einer bevorzugten Ausführung einer Tablettenteststation beschrieben, die Länge, Breite und Härte, also Bruchfestigkeit und/oder Abdeckelverhalten prüft.

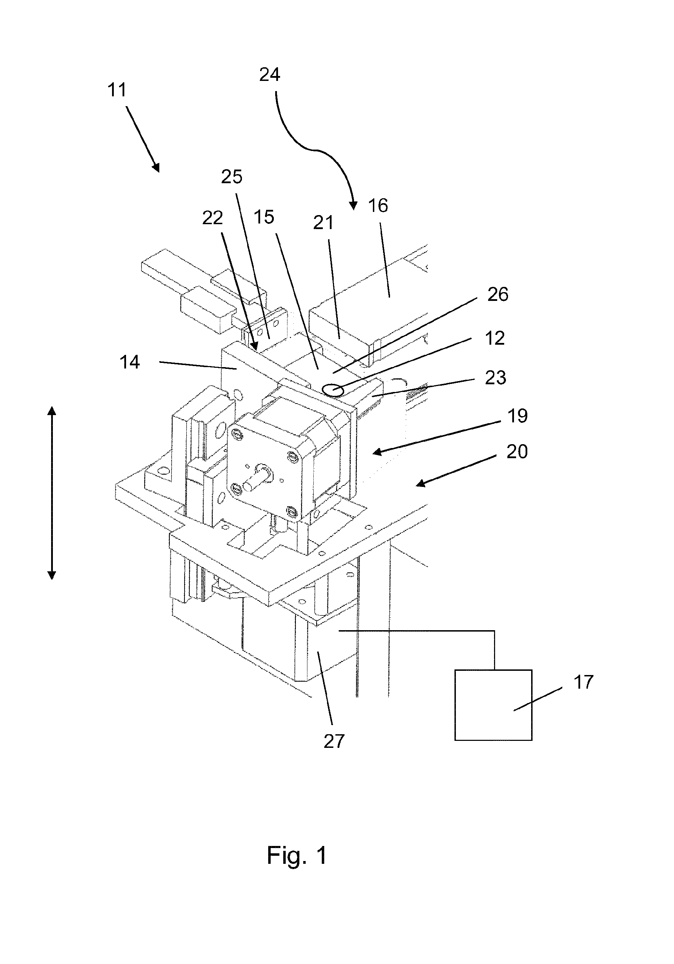

Die Figur 1 zeigt eine Tablettenteststation 11 mit einer Hebevorrichtung 20, deren Steuerung 17 dem Antriebsmittel, insbesondere Motor 27 - denkbar wäre aber auch ein anderes Mittel, bspw. ein pneumatisches - der Hebevorrichtung 20 Positionen für die Aufnahme 15 vorgibt, in die sie zur Übergabe von der Beschickungsvorrichtung oder zur Durchführung eines Verfahrensschrittes beim Prüfen, Testen und/oder Messen einer Tablette verfahren und in denen sie während des jeweiligen Verfahrensschrittes gehalten wird.

Die Aufnahme 15 für eine Tablette 12 ist hier beweglich, als Klappe mit Positionierfläche 26 und einer Umlenkung 23 ausgebildet. Sie ist mit ihrem separaten Antrieb 19 gezeigt, mit dem sie gemeinsam durch die Hebevorrichtung 20 höhenverfahren wird. Es ist eine Bruchvorrichtung 24 vorgesehen, die eine fixe Bruchbacke 14 und eine verschiebbare Bruchbacke 16 umfasst. Die fixe Bruchbacke 14 weist eine Positionierfläche 22 auf. Die verschiebbare Bruchbacke 16 weist eine Positionierfläche 21 auf.

Die verschiebbare Bruchbacke 16 wird sowohl zum Positionieren der Tablette 12 für das oder die Messverfahren als auch zum Bruchfestigkeitstest an der Tablette 12, beziehungsweise zur Überprüfung des Bruchverhaltens über die Aufnahme 15 in Richtung der fixen Bruchbacke 14 verschoben, wenn die Hebevorrichtung 20 abgesenkt ist, wie hier dargestellt. Dabei kommt die Positionierfläche 21 in Anlage zur Tablette 12 und schiebt diese - sofern die Tablette nicht wie meistens angestrebt bereits an die fixe Bruchbacke zu liegen kommt -, bis sie zur Anlage an Positionierfläche 22 kommt und rechtwinklig zur fixen Bruchbacke 14 ausgerichtet ist. Daran anschliessend wird mittels einer Messvorrichtung 25 eine Breiten- , Längen- und/oder Dickenmessung an der Tablette vorgenommen. Anschliessend wird die Tablette 12 vorzugsweise durch mindestens eine Schwenkbewegung der Aufnahme 15 oder - im Falle der Zweiteiligkeit der Aufnahme 15 - durch eine Schwenkbewegung der Aufnahmeflächen gegen einander zu einer V-Stellung in eine senkrechte Position relativ zur fixen Bruchbacke 14 gebracht. Danach wird der Bruchfestigkeits-, beziehungsweise Bruchverhaltenstest durchgeführt, in dem die Positionierfläche 21 der verschiebbaren Bruchbacke die Tablette 12 gegen die fixe Bruchbacke 14 drückt, wobei sie gegebenenfalls zerbricht. Anschliessend wird die Tablette 12 oder ihre Bruchteile vorzugsweise durch ein Verschwenken der Aufnahme 15 aus der Tablettenteststation 11 hinausbefördert.

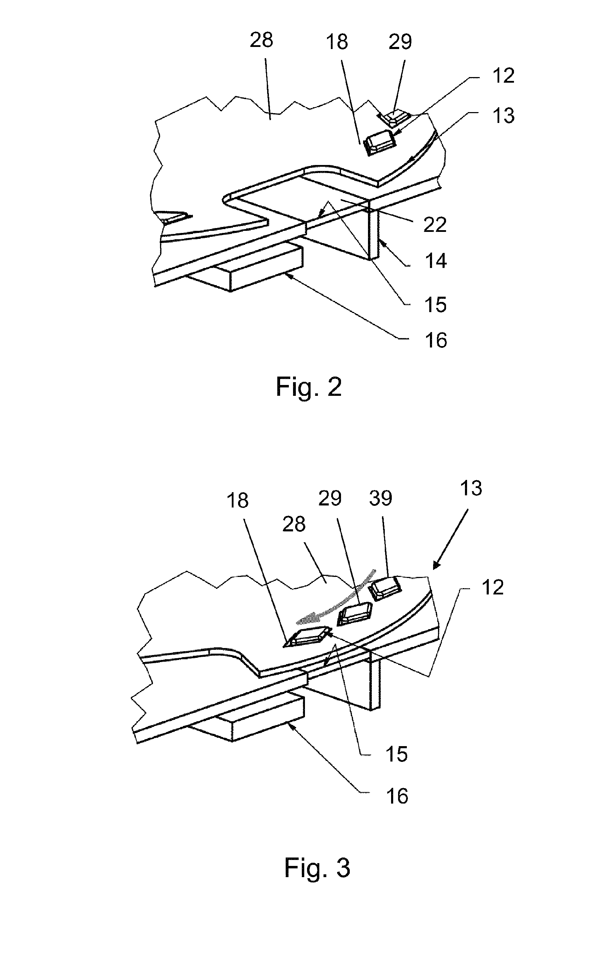

Die Figuren 2 bis 5 zeigen verschiedene Phasen des, beziehungsweise der oben beschriebenen Testverfahren. Besonderes Augenmerk ist hierbei auf die Beschickungsvorrichtung gerichtet. Im Prinzip kann die Tablettenübergabe mittels jeder bekannten Beschickungsvorrichtung geschehen. Besonders vorteilhaft ist die beschriebene Hebevorrichtung jedoch für Beschickungsvorrichtungen, deren Übergabestelle, also Auslass oder dergleichen, konstruktiv bedingt, bei der Beschickung relativ weit von der Fläche entfernt ist, die die Tablette für zumindest das erste Prüfverfahren aufnehmen soll. Je grösser nämlich der Abstand zwischen dem Auslass der Beschickungsvorrichtung und der Aufnahme für die Tablette ist, desto grösser ist die Gefahr, dass die Tablette unkontrolliert auf die Aufnahme fällt oder rutscht und somit an einer für das anschliessende Prüfverfahren ungünstigen Stelle zu liegen kommt. Für bestimmte Tablettenformen ist jedoch eine bestimmte Vororientierung der Tablette auf der Aufnahme für ein brauchbares Testergebnis zwingend.

Figur 2 zeigt den Transport einer Tablette 12 zur Aufnahme 15 mittels einer Beschickungsvorrichtung 13. Die Beschickungsvorrichtung 13 ist in einer bevorzugten Ausführung als Drehteller 28 dargestellt. Es liesse sich aber ebenso gut jede andere Art vorzugsweise automatischer Beschickungsvorrichtung denken, bspw. ein Linearförderer. Der Drehteller 28 ist in den Figuren 2 bis 5 beispielhaft jeweils mit mehreren rechteckigen Auslässen 18, 29, 39 dargestellt, welche als Ausnehmungen ausgebildet sind. Selbstredend ist auch jede andere zweckdienliche Form der Auslässe 18, 29, 39 denkbar. In einer bevorzugten Ausführung hat der Drehteller 28 Auslässe, die so geformt sind, dass sie geeignet sind, verschiedene Tabletten in der für das nachfolgende Verfahren idealen Position abzulegen. Während die Beschickungsvorrichtung 13 die (jeweils nächste) Tablette 12 zur Aufnahme befördert, ist die Hebevorrichtung 20 (hier nicht gezeigt) in einer Höhe gehalten, die die kleinstmögliche Beabstandung der Auflage 15 von der Beschickungsvorrichtung 13 zulässt. Die verschiebbare Bruchbacke 16 ist in einem Abstand zur fixen Bruchbacke 14 gehalten, der dem Hebeaggregat 20 ermöglicht, die Auflage 15 in einem späteren Schritt zwischen beiden Bruchbacken 14 und 16 hindurch nach unten zu verfahren.

Die Figur 3 ist der Drehteller 28 so verfahren, dass der Auslass 18 des Drehtellers 28 so über der Aufnahme 15 liegt, dass die Tablette 12 an der für sie vordefinierten Position abgelegt wird. Hebevorrichtung 20 und Bruchbacke 16 befinden sich noch an der gleichen Position wie in Figur 2.

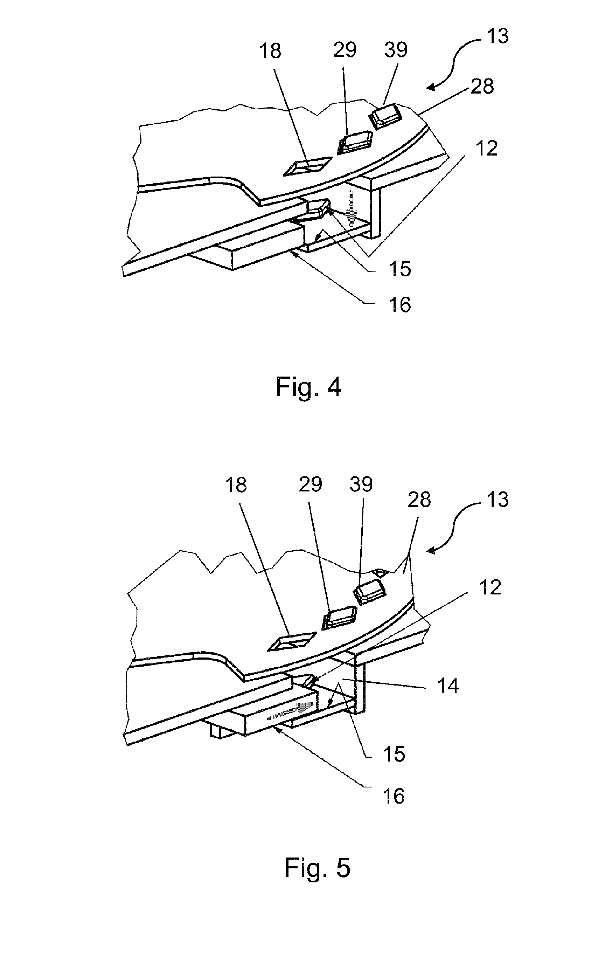

In Figur 4 ist nun die Tablette 12 auf der Aufnahme 15 abgelegt und der Auslass 18 wieder frei zu sehen. Die Hebevorrichtung 20 hat die Aufnahme 15 relativ zur Beschickungsvorrichtung 13 so weit nach unten verfahren, dass ein Testverfahren, beispielsweise ein Verfahren zur Ermittlung von Länge, Breite oder Dicke auf der konstruktionsbedingten Höhe der Messaggregate innerhalb der Tablettenteststation 11 durchgeführt und die Tablette 12 je entsprechend positioniert werden kann.

Figur 5 zeigt wie die verschiebbare Bruchbacke 16 die Tablette 12 zur Bruchfestigkeitsprüfung in Richtung auf die fixe Bruchbacke 14 verschoben hat oder die Tablette bereits ohne Verschiebung so positioniert werden konnte. Die Hebevorrichtung 20 hält die Aufnahme 15 in der gleichen, unteren Position wie in Figur 4 gezeigt. Ist die Tablette 12 abschliessend aus der Tablettenteststation 11 ausgeschieden worden, fährt die Hebevorrichtung 20 wieder relativ zur Beschickungsvorrichtung 13 nach oben wie in Figur 2 beschrieben.

Die einzelnen, im Zusammenhang mit den Figuren beschriebenen Details können selbstverständlich auch bei den anderen Ausführungsformen im Rahmen der Patentansprüche vorgesehen werden.

### Bezugszeichenliste

- 11: Tablettenteststation
- 12: Tablette
- 13: Beschickungsvorrichtung
- 14: fixe Bruchbacke
- 15: Aufnahme für Tablette
- 16: verschiebbare Bruchbacke
- 17: Steuerung
- 18: Auslass
- 19: Antrieb für Aufnahme und Aufhängung desselben
- 20: Hebevorrichtung
- 21: Positionierfläche der verschiebbaren Bruchbacke
- 22: Positionierfläche der fixen Bruchbacke
- 23: Umlenkung
- 24: Bruchvorrichtung
- 25: optische Messvorrichtung
- 26: Positionierfläche der Aufnahme
- 27: Antrieb, insbesondere Motor der Hebevorrichtung
- 28: Drehteller
- 29, 39: Auslass

## Patentansprüche

1. Tablettenteststation, umfassend
mindestens eine Aufnahme zur Übernahme von Tabletten von einem Auslass einer Beschickungsvorrichtung und
mindestens ein Prüfmittel zum Prüfen, Testen und/oder Messen der Tabletten, **dadurch gekennzeichnet, dass**
eine Hebevorrichtung (20) zum Verfahren der Aufnahme (15) relativ zur Beschickungseinrichtung (13) vorgesehen ist.

2. Tablettenteststation nach Anspruch 1,**dadurch gekennzeichnet, dass** die Hebevorrichtung (29) ein Antriebsmittel, insbesondere einen Motor (27) umfasst.

3. Tablettenteststation nach Anspruch 1 oder 2, **dadurch gekennzeichnet, dass** die Hebevorrichtung eine Steuerung (17) insbesondere für das Antriebsmittel, insbesondere Motor (27), umfasst.

4. Tablettenteststation nach einem der Ansprüche 1-3, **dadurch gekennzeichnet, dass** das Mittel zum Prüfen, Testen und/oder Messen der Tabletten als Längen- und/oder Breiten- und/oder Dickenmessvorrichtung ausgebildet ist, die vorzugsweise mindestens eine Positionierfläche zum Positionieren der Tablette (12) auf der Aufnahme (15) aufweist.

5. Tablettenteststation nach Anspruch 4, **dadurch gekennzeichnet, dass** das Mittel zum Prüfen, Testen und/oder Messen der Tabletten ein optisches (25) ist.

6. Tablettenteststation nach einem der Ansprüche 1-3, **dadurch gekennzeichnet, dass** das Mittel zum Prüfen, Testen und/oder Messen der Tabletten als Bruchfestigkeitsmessvorrichtung und/oder Vorrichtung zum Testen des Bruchverhaltens ausgebildet ist.

7. Tablettenteststation nach Anspruch 6, **dadurch gekennzeichnet, dass** das Mittel zum Prüfen, Testen und/oder Messen der Tabletten eine Bruchvorrichtung (24) umfasst, die mindestens eine Positionierfläche (21, 22) zum Positionieren der Tablette (12) aufweist.

8. Tablettenteststation nach einem der vorangehenden Ansprüche, **dadurch gekennzeichnet, dass** die Aufnahme (15) mindestens eine Positionierfläche (23, 26) zum Positionieren der Tablette (12) aufweist.

9. Tablettenteststation nach Anspruch 8, **dadurch gekennzeichnet, dass** die Positionierfläche der Aufnahme (15) als Umlenkung (23) ausgebildet ist.

10. Tablettenteststation nach Anspruch 8, **dadurch gekennzeichnet, dass** die Aufnahme (15) als mindestens eine Klappe ausgebildet ist.

11. Tablettenteststation nach einem der vorangehenden Ansprüche, **dadurch gekennzeichnet, dass** die Mittel zum Prüfen, Testen und/oder Messen der Tabletten in einer Ebene angeordnet sind.

12. Maschine mit einer Tablettenteststation nach einem der vorangehenden Ansprüche.

13. Verfahren zum Testen von Tabletten mit einer Tablettenteststation nach Anspruch 1 bis 11 oder mit einer Maschine nach Anspruch 12, umfassend Verfahren der Aufnahme (15) zur Übergabe der Tablette (12) mittels der Hebevorrichtung (20) in eine Position benachbart der Beschickungsvorrichtung (13),
Übergabe der Tablette (12) von der Beschickungsvorrichtung (13) an die Aufnahme (15),
anschliessend Verfahren der Aufnahme (15) in mindestens eine zweite Position zur Durchführung von Tests.

14. Verfahren zum Testen von Tabletten nach Anspruch 13, **dadurch gekennzeichnet, dass** die Hebevorrichtung (20) die Aufnahme (15) zur Übergabe der Tablette (12) so in die erste Position verfährt, dass der Abstand der Aufnahme (15) vom Auslass (18) der Beschickungsvorrichtung (13) kleiner als die Dicke der jeweils zu prüfenden Tablette (12) ist.

15. Verfahren zum Testen von Tabletten nach Anspruch 13 oder 14, **dadurch gekennzeichnet, dass** die Tablette (12) zum Abschluss des Verfahrens aus der Tablettenteststation (11) ausgeschieden wird.
